# EUROPEAN PATENT APPLICATION

(11) **EP 1 932 518 A1**
(43) Date of publication of application: **18.06.2008**
(21) Application number: 06077206.8
(22) Date of filing: 11.12.2006
(51) Int. Cl.: A61K 9/127, A61K 31/22, A61P 35/00

(54) **Statin containing compositions for treatment of cancer**

(71) Applicant: Universiteit Utrecht Holding B.V., 3584 CM Utrecht (NL)
(72) Inventor: The designation of the inventor has not yet been filed
(74) Representative: van Loon, C.J.J.

(57) **Abstract**

The present invention relates to compositions comprising statin, and especially to the use of such compositions in the treatment of cancer or in the inhibition of cancer growth. More specifically, the invention relates to a method for targeting a statin to tumor tissue.

## Description

The present invention relates to compositions comprising statin, and especially to the use of such compositions in the treatment of cancer or in the inhibition of cancer growth. More specifically, the invention relates to a method for targeting a statin to tumor tissue. Even more specifically, the present invention aims to provide the use of such compositions in the treatment of non-lymphatic cancers, and preferably solid (non-hematological) malignancies and metastases (solid primary and secondary tumors), and is hence in the field of oncology. In the treatment of non-solid tumors or lymphatic cancer types, e.g. chronic and acute lymphatic leukaemia, Hodgkin and non-Hodgkin lymphomas, statins can however also be used.

Statins are well-known lipid lowering agents. Particularly, drugs of the statin-class are structurally similar to hydroxymethylglutaryl-coenzyme A (HMG-CoA,) a precursor of cholesterol, and, as such, these drugs are competitive inhibitors of HMG-CoA reductase. This latter enzyme regulates the rate-limiting step in the synthesis of cholesterol. The inhibition of this enzyme results in a decrease in intracellular cholesterol level, which leads to an upregulation of LDL-receptor activity and a reduction of the entry of LDL into the circulation.

Multiple clinical trials using statins have demonstrated a significant improvement in cardiovascular end points. An additional important observation is that their beneficial effects on clinical outcomes considerably exceed the influence on regression of coronary stenosis. This discrepancy between clinical and angiographic effects appears attributable to additional properties other than their lipid lowering potential.

Several studies have addressed non-lipid mechanisms of statin action that may contribute to the cardiovascular event reduction. These extra effects have been suggested to involve alterations of isoprenoid levels.

Studies investigating additional mechanisms of statin action demonstrated improvements in maintenance of endothelial function, tissue antioxidant capacity, inhibition of smooth muscle cell proliferation and inflammation. Furthermore, statins may influence thrombosis through variable inhibitory actions on platelet function, coagulation factors, rheology and fibrinolysis. (Kurian KC, Rai P, Sankaran S, Jacob B, Chiong J, Miller AB. The effect of statins in heart failure: beyond its cholesterol-lowering effect. J Card Fail. 2006 12(6):473-8).

Recently, Rutishauser in Swiss Med Wkly, (2006) 136 (3-4): 41-49 supplemented the effects of these agents by evidence showing that statins may affect the function of immune and inflammatory cells.

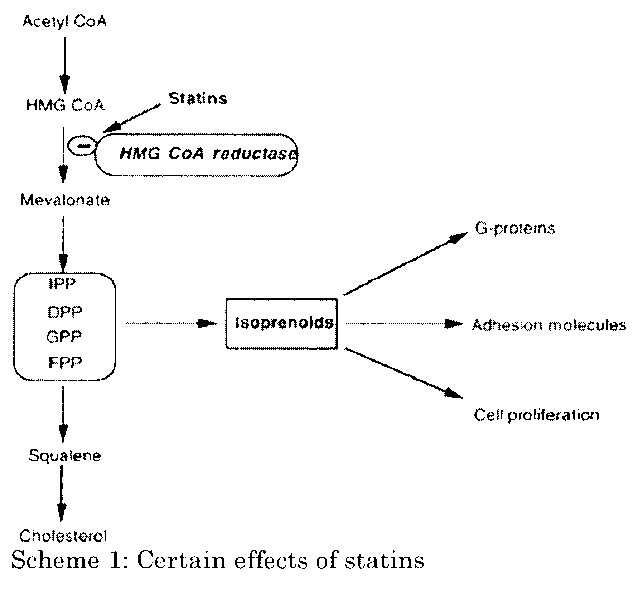

Statins have also been suggested to have effects on cancer growth (see e.g. Lam & Cao, Drugs Today (Barc), (2000) 41(5), 329-334). However, the data on these effects are inconsistent.

Some evidence (see Fritz, Int J Oncol (2005) 27(5), 1401-1409) suggests that statins may reduce cancer incidence and also cancer progression, but the mechanisms by which this occurs is unknown. The hypothesis that inflammatory processes in and around the tumor are important in the angiogenic cascade suggests that statins' immunosuppressive action may also be involved.

Other evidence, however suggests the opposite and statins have also been shown to promote angiogenesis which could promote cancer growth. In this light, reference is made to Walter et al., Coron Artery Dis. (2004) 15(5): 235-242.

These conflicting studies indicate that the effects of statins are not clear-cut and that additional measures are needed to predictably exploit the full potential of statins as anticancer therapeutics.

In addition, the amounts of statin required to obtain any effect, if at all, are high. That is, if one were to use statins in cancer- or tumor-therapy, one would need high dosing (typical pre-clinical doses are 50-150 mg/kg per week (See, *e.g.* Shibata et al. Carcinogenesis. 25(10) (2004):1887-1898.) for prolonged periods of time to obtain significant tumor growth inhibition. This would intensify all kinds of side-effects and will most likely cause toxicities to arise.

The present invention focuses on the aspect of providing measures to predictably exploit the full potential of statins. It is, hence, an objective of the present invention to find a method to target statins to tumor tissue for or in the treatment, retardation or inhibition of cancer.

In accordance with the present invention, it has been found that by selectively delivering statins to tumor tissue so that local drug concentrations are increased, a pharmacological effect towards cell types involved in the progression of cancer is achieved.

In a first aspect, the present invention relates to the use of statins, encapsulated in colloidal carriers in the manufacture of a medicament useful in the treatment of cancer. Preferably, the medicament is used in the treatment of non-lymphatic cancer, and especially in the treatment of solid primary and/or secondary tumors. The medicament of the present invention can however also be used in the treatment of lymphatic cancers.

In a second aspect, the present invention relates to statins, encapsulated in colloidal carriers.

Not only makes the invention the effects of statins predictable, but it also reduces the overall dose required to obtain the desired effects. Further, in accordance with the present invention, an increased tumor specificity and, hence, a decreased likelihood of side effects is obtained. This is important as the doses needed to observe the anti-cancer effects for the free drugs are comparatively high, which would intensify side-effects and could cause new toxicities to arise.

As mentioned herein-above, the present invention is directed to the use of encapsulated statins. Statins, including all HMG-CoA reductase inhibitors, form a class of hypolipidemic agents, used as pharmaceuticals to lower cholesterol levels in people with or at risk for cardiovascular disease. They cause cholesterol lowering by inhibiting the enzyme HMG-CoA reductase, an enzyme involved in the rate of cholesterol synthesis. Inhibition of this enzyme in the liver stimulates the LDL-receptors, which results in an increased clearance of LDL from the bloodstream and a decrease in blood cholesterol levels. The said encapsulated statins were found to inhibit tumor growth, especially solid tumor growth, and can hence be used for this effect in the treatment of cancer, and especially in oncology.

In a preferred embodiment of the present invention, the statin is selected from the group consisting of atorvastatin, ceruvastatin, fluvastatin, lovastatin, pravastatin, pitavastatin, simvastatin, rosuvastatin and chemical derivatives thereof, including the pharmaceutical effective salts, solvates, esters and adducts thereof.

Structural formulas of the preferred statins are shown here-after:

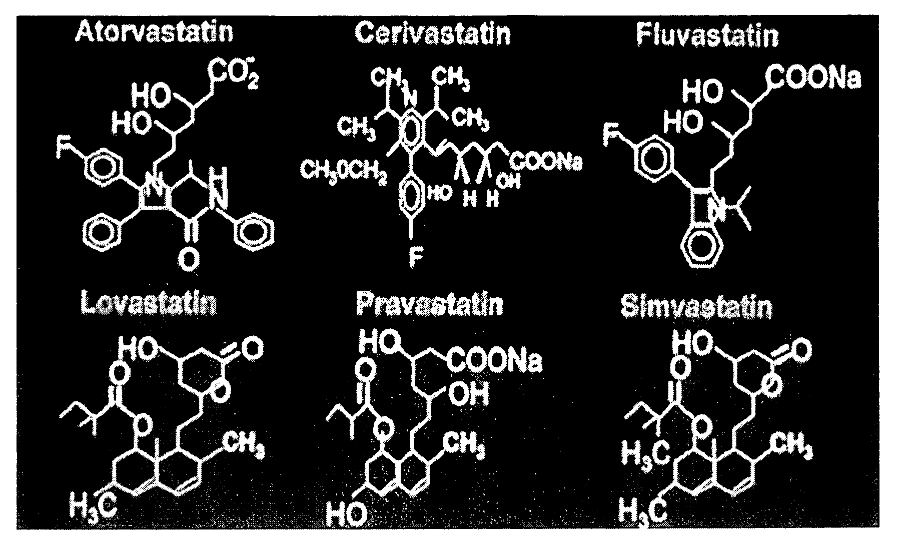

In order to be effective, the statins are encapsulated in colloidal carriers. Such carriers should have a selected mean particle diameter in the size range between about 10-400 nm (preferably between 40 and 200 nm in diameter), giving an increased localization and improved retention of the encapsulated compound at the tumor site. The carrier system provides stable encapsulation and provides protection from degradation. Examples of such colloidal carrier system are long-circulating liposomes, lipoproteins, lipid micelles, polymeric micelles, nanoparticles and nanocapsules, which carriers should be long-circulating. Long-circulating carriers preferably have a circulation half-life of at least 3 hours, and especially at least 6 hours. The circulation half-life is, as the person skilled in the art appreciates, defined as the time at which the second linear phase of the logarithmic carrier, for instance liposomal, clearance profile reaches 50% of its initial concentration, which is the extrapolated plasma concentration at t=0.

Suitable long-circulating colloidal carriers have very favorable pharmacokinetics, a favorable tissue distribution behavior and an efficient half life. Additionally, a stable association between statin and the carrier system is observed, while the loading with statin is efficient. Further, a good biological availability at the site where activity is required is observed.

Without wishing to be bound by any theory, it is hypothesized that the colloidal carriers have an interaction with macrophages in the tumor. This would mean that said cell type could be down regulated in tumor therapy, or that it may liberate the encapsulated statins to allow interaction with other cell types in and around the tumor. In addition, it is hypothesized that long-circulating carriers used in the present invention accumulate at sites of malignant tissues such as tumors as a result of the enhanced permeability of tumor vasculature as compared to healthy endothelium, allowing an improved localization and improved retention of the statin at these sites.

In a preferred embodiment the long-circulating colloidal carrier is a liposome, a nanocapsule or a polymeric micelle; especially one that has a neutral or negative charge at physiological conditions.

Other suitable long-circulating carriers can be based on lipoproteins, and especially high density lipoproteins and low density lipoproteins, and on lipoprotein mimetics or neo-lipoproteins. It has been found that long-circulating microvesicles, and especially long-circulating liposomes, nanocapsules and polymeric micelles, are capable of efficiently delivering statin drugs to a specific site, i.e. to a tumor.

The long-circulating colloidal carriers used in accordance with the present invention typically have a mean particle diameter of less than 450, and preferably less than 300 nm as determined by Dynamic light scattering using a Malvern 4700™ system equipped with a He/Ne laser, and preferably of about 40 - 200 nm.

In the most preferred embodiment, the colloidal carrier is formed by long-circulating liposomes comprising a non-charged vesicle-forming lipid, 0-20 mole percent of an amphipathic vesicle-forming lipid derivatised with polyethyleneglycol, 0-50 mole percent of a sterol, and 0-10 mol % of a negatively charged vesicle-forming lipid, which liposomes have a selected mean particle diameter in the size range between about 40 - 200 nm.

Such long-circulating liposomes are already known in the art. More particularly, these known liposome systems are described to be useful in site-specific treatment of inflammatory disorders in WO-A-02/45688. For the preparation of suitable compositions to be used in the present invention, the preparation methods described in said WO-A-02/45688 are incorporated herein by reference. In this document WO-A-02/45688, the liposome systems described in EP-A-0 662 820 are adapted to become "long-circulating".

EP-A-1 044 679 relates to liposomes having a drug included therein, which are said to have an ensured stability in blood. In addition these liposomes have an active targeting property to proteoglycan-rich areas. These areas are created because with some diseases, an over-production of proteoglycans occurs; said proteoglycans keeping cell surfaces anionic. To target liposomes to these anionic surfaces, the liposomes need to be cationic in nature. Thereto, the said liposomes require the presence of a basic compound taking positive charge within a physiological pH range.

The liposomes of the present invention do not require the active targeting property described in EP-A-1 044 679. That is, no specific homing groups are required to selectively bring the colloidal carriers containing statins to the tumor sites. However, to increase the selectivity to an even higher extent, it is possible to attach or incorporate tumor specific antibodies or receptor ligands or food compounds at the outside surface of the carrier molecules so as to increase the interaction possibilities with the tumor cells or the cells in or around the tumor. The "targeting" of the neutral or optionally negatively charged liposomes of the present invention is ruled by the above-identified increased permeability in the tumor vasculature. That is, the present invention is for a major part based on passive accumulation, rather than active targeting.

The colloidal carriers, such as the liposomes useful in the present invention should not have a positive charge and should hence not comprise components that give the carriers a positive charge at physiological pH; that is at physiological pH, being a pH of between 6 and 8, the overall charge of the carrier to be used in the present invention should be neutral or negatively charged. Preferred liposomes are based on non-charged vesicle-forming lipids. Neutral or non-charged vesicle-forming lipids lead to a suitable long circulation time.

Typically, 5-10 mole% of negatively charged lipids may be present. Preferred lipids to be used to prepare the microvesicles used in the invention comprise saturated phospholipids and sphingolipids in combination with sterols, such as cholesterol and/or ergosterol and derivatives thereof. Substitution (complete or partial) of these basic components by e.g. sphingomyelines appeared to be possible.

To secure a suitable stability in the blood circulation system 10-50 mole % sterols should be present in the microvesicle material. Suitable liposome constituents are described in the above-identified WO-A-02/45688 and EP-A-0 662 820. More preferably, the liposomes contain at least one type of polymer lipid conjugates, such as lipids derivatised with polyalkylene glycol, preferably with polyethylene glycol (PEG). Suitable polymer-lipid-conjugates have a molecular weight of between 200 and 30,000 Dalton.

Other suitable candidates to be used in these polymer-lipid-conjugates or water-soluble polymers such as: poly ((derivatized) carbohydrate)s, water-soluble vinylpolymers (e.g. poly(vinylpyrrolidone), polyacrylamide and poly(acryloylmorpholine) and poly(methyl/ethyl oxazone). These polymers are coupled to the lipid through conventional anchoring molecules. Suitably, the concentration of polymer lipid conjugates is 0-20 mole%, and preferably 1-10 mole%, based upon the total molar ratio of the vesicle forming lipids. The presence of these polymer-lipid-conjugates has a favorable effect on the circulation time. However, by carefully selecting specific lipid compositions an physical specifications suitable long circulation times can be obtained without using a polymer-lipid-conjugate; for example, 50-100 nm liposomes of distearylphopshatidylcholine and cholesterol and/or sphingolipids like sphingomyelin. The liposomes may additionally contain one or more types of charged vesicle-forming lipids, e.g. phosphatidylglycerol, phosphatidylethanolamine, (di)stearylamine, phosphatidylserine, dioleoyl trimethylammonium propane, phosphatidic acids and cholesterol hemisuccinate. Typically, the concentration of charged vesicle-forming lipids is 0-15 mole%, preferably 0-10 mole% based upon the molar ratio of the vesicle forming lipids.

Polymeric micelles to be used in the present invention can be made in accordance with the method described in EP-A-1 072 617 adapted in accordance with the above-described method for the preparation of liposomes.

Passive loading of the active ingredients into the liposomes by dissolving the statins in the aqueous phase is sufficient in order to reach an encapsulation as high as possible, but other methods can also be used.

Where in this description reference is made to charged/uncharged/amphiphatic, and so on, this reference relates to physiological conditions.

As said, the present invention also relates to pharmaceutical compositions comprising statins, encapsulated in colloidal carriers, as defined herein-above. That is, the present invention provides a medicament for or in the treatment of cancer, suitable to administer statins in relatively low dosages. Suitably, the medicament is a medicament for parental or local application. Application through the oral or pulmonal route are however also possible.

In accordance with the invention, effective inhibitions in tumor growth have been observed in particular embodiments with relatively low dosages, less than 45, preferably less than 30, such as at least 5, for eample about only 10 mg/kg body weight per week.

A composition used in accordance with the present invention may comprise one or more additional components:

Compositions to be used in accordance with the present invention may suitably contain or comprise at least one compound selected from the group consisting of cytostatic agents and cytotoxic agents, preferably at least one compound selected from the group consisting of doxorubicin and taxol.

Moreover, suitable use can be made of compositions comprising at least one component selected from the group consisting of immunomodulators and immunosuppressants. Examples of such components are methotrexate, cyclophosphamide, cyclosporin, muramyl peptides, cytokines and penicillamine.

The present invention will now be described in more detail, while referring to the following examples. In the examples, reference will be made to figures, wherein:

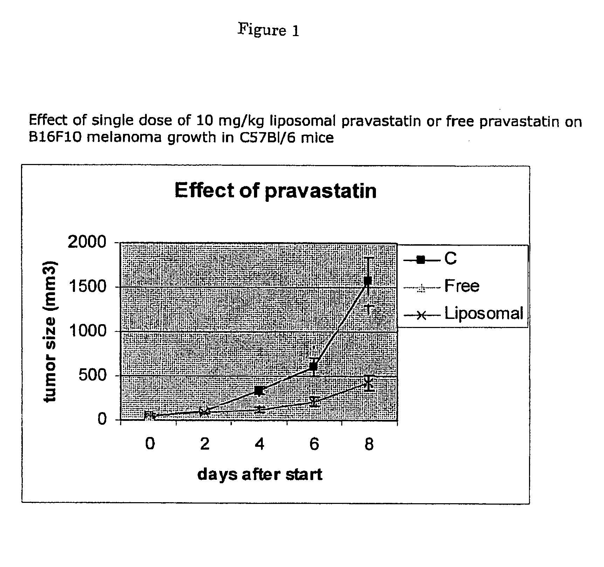

Figure 1 showing the effect of parvastatin on tumor size, and especially the effect of a single dose of 10 mg/kg liposomal pravastatin or free pravastatin on B16F10 melanoma growth in C57B1/6 mice.

Any percentages mentioned are percentages by weight drawn to the final composition, unless otherwise indicated.

### Example 1 - Liposome preparation

Long-circulating liposomes were prepared by dissolving dipalmitoylphosphatidylcholine (DPPC) (Lipoid GmbH, Ludwigshafen, Germany), cholesterol (Chol) (Sigma, St. Louis, USA), and poly(ethylene) glycol 2000-distearoylphosphatidylethanolamine (PEG-DSPE) (Lipoid GmbH) in a molar ratio of 1.85:1.0:0.15, respectively, in chloroform:methanol (2:1 vol:vol) in a round-bottom flask. Typically batch sizes of 1000-2000 µmol total lipid were used.

Liposomes were formed by addition of an aqueous solution of 10 mg/ml pravastatin (Sigma-Aldrich) The water-soluble statin was used to ensure stable encapsulation in the liposomes. In case of labeling of the liposomes with ¹¹¹In-oxine (Mallinckrodt Medical, Petten, The Netherlands), liposomes were formed by addition of 5 mM DTPA/10 mM Hepes/135 mM NaCl-buffer pH 7 to the lipid film, according to a procedure described by Boerman et al. in J. Nucl. Med. 36(9) (1995), 1639-1644. Liposome size was reduced by multiple extrusion steps through polycarbonate membranes (Nuclepore, Pleasanton, USA) with a final pore size of 50 nm.

The mean particle size of the long-circulating liposomes was determined by dynamic light scattering to be 0.1 µm with a polydispersity value of approximately 0.1. The polydispersity value varies between 0 and 1. A value of 1 indicates large variations in particle size, whereas a value of 0 indicates a complete monodisperse system. Thus, the present preparations showed limited variation in particle size. The amount of lipid in the liposome dispersion was determined by colorimetric phosphate determination according to Rouser (Lipids 5 (1970), 494-496).

The concentration of pravastatin in the liposomes was determined by spectrophotometric absorbance at 238 nm; samples were measured on a Perkin Elmer UV-VIS spectrophotometer in ethanol using dissolved lipids in ethanol as controls.

### Example 2 - Tissue distribution of liposomes

B16 murine melanoma or C26 colon carcinoma cells were cultured at 37°C in a 5% CO₂-containing humidified atmosphere in DMEM medium (Gibco, Breda, The Netherlands) containing 10% (v/v) fetal calf serum supplemented with 2mM L-glutamine, 100 IU/ml penicillin, 100zg/ml streptomycin and 0.25g/ml amphotericin B (Gibco).

Male C57B1/6 mice or male Balb/c mice (6-8 weeks of age) were obtained from Charles River, kept in standard housing with standard rodent chow and water available ad libitum, and a 12 h light/dark cycle. Experiments were performed according to national regulations and approved by the local animal experiments ethical committee. For tumor induction,1 x 10⁶ B16 melanoma cells were inoculated subcutaneously in the flank of syngeneic C57BI/6 mice, whereas 1 x 10⁶ C26 tumor cells were inoculated in Balb/c mice.

The tissue distribution of ¹¹¹In-labeled PEG-liposomes according to Example 1 in tumor bearing mice was determined as follows. At a tumor volume of approximately 1 cm³, mice were injected i.v. with 25 µmol lipid/kg (corresponding to 30 x106 cpm/mouse) of ¹¹¹In-labeled liposomes. At 6 h and 24 h after injection animals were sacrificed, a blood sample was taken and tumor, lung, liver, spleen and kidneys were dissected, weighed and radioactivity was counted. The results are shown in Table 1.

**Table 1. Tissue distribution of long-circulating liposomes at 6 h and 24 h after intravenous administration in s.c. C26 colon carcinoma or s.c. B16F10 melanoma bearing Balb/c and C57BI/6 mice, respectively. Tumors weighed approximately 1 g. Values represent % of injected dose/ g tissue.**

| **Organ** | **C26 colon carcinoma** | | | **B16F10 melanoma** | |
|---|---|---|---|---|---|
| | **6h** | **24h** | | **6h** | **24h** |
| **blood** | 21.9±4.5 | 7.1±2.7 | | 24.2±2.3 | 6.3±0.7 |
| **tumor** | 5.1±1.9 | 7.4±2.2 | | 5.3±2.9 | 5.8±1.5 |
| **lung** | 9.5±2.7 | 3.6±0.6 | | 12.1±0.7 | 3.2±1.3 |
| **spleen** | 23.8±11. 3 | 25.9±10. 1 | | 29.6±6.7 | 29.8±10.8 |
| **kidney** | 10.1±2.1 | 8.6±1.9 | | 9.8±3.3 | 8.0±3.0 |
| **liver** | 8.7±2.1 | 14.0±4.4 | | 8.7±3.6 | 13.4±5.6 |
| **tumor/ blood ratio** | 0.2±0.1 | 1.0±0.3 | | 0.2±0.1 | 0.9±0.2 |

| | | | | | |
|---|---|---|---|---|---|
| Mean ± S.D. N=5 animals/experimental group. | | | | | |

15% of the injected dose of radioactively labeled liposomes was still present in the circulation in both mouse models at 24 h after injection.

Approximately 5-10% of the injected dose could be recovered from tumor tissue in both models. Approximately the same amount was present in the livers. Relatively low amounts of liposomes were recovered from spleen, kidney and lungs.

### Example 3 - Tumor growth inhibition

B16F10 melanoma bearing mice received a single intravenous injection of an indicated dose of free pravastatin or liposomal pravastatin at the time when the tumor measured 50 mm³. At 7 days after treatment, tumor size was measured and tumor volume calculated according to the equation V = 0.52 x a² x b, wherein a is the smallest and b is the largest superficial diameter.

To compare the effect of different doses of free pravastatin or liposomal pravastatin on tumor growth, B16-tumor bearing mice received a single injection of either formulation at the moment that the tumor became palpable. At 1 week after injection tumor volume was smaller with liposomal pravastatin. See in this light Figure 1, wherein curve C refers to an untreated control.

## Claims

1. Use of statins, encapsulated in colloidal carriers in the manufacture of a medicament useful in the treatment of cancer.

2. The use of claim 1, wherein the medicament is useful in the treatment of non-lymphatic cancer, and especially in the treatment of solid primary and/or secondary tumors.

3. The use of claim 1 or 2, wherein the statin is selected from the group consisting of atorvastatin, pitavastatin, ceruvastatin, fluvastatin, lovastatin, pravastatin, simvastatin, rosuvastatin and derivatives thereof.

4. The use according to any one of the preceding claims, wherein the microvesicle is a liposome, nano-capsule or a polymeric micelle, which microvesicle has a neutral or negative charge at physiological conditions.

5. The use according to any one of the preceding claims, wherein the microvesicles are liposomes comprising a non-charged vesicle-forming lipid, 0-20 mole percent of an amphipathic vesicle-forming lipid derivatised with polyethyleneglycol, 0-50 mole percent of a sterol, and 0-10 mol % of a negatively charged vesicle-forming lipid, which liposomes have a selected mean particle diameter in the size range between about 40 - 200 nm.

6. The use according to any one of the preceding claims, wherein the microvesicle has a circulation half-life of at least 6 hours.

7. The use according to any one of the preceding claims, wherein the medicament is a medicament for parental or local application.

8. Pharmaceutical composition comprising statins, encapsulated in colloidal carriers, as defined in any one of the preceding claims.
